# EUROPEAN PATENT APPLICATION

(11) **EP 1 340 518 A2**
(43) Date of publication of application: **03.09.2003**
(21) Application number: 03075582.1
(22) Date of filing: 26.02.2003
(51) Int. Cl.: A61M 37/00

(54) **Combined transponder and injector**

(30) Priority: 28.02.2002 US 361900 P
(71) Applicant: Datamars SA, 6930 Bedano-Lugano (CH)
(72) Inventor: Malfanti, Roberto, 6968 Sonvico (CH); Oggian, Sileno, 6930 Bedano (CH)
(74) Representative: Carpintero Lopez, Francisco

(57) **Abstract**

Apparatus for implanting a transponder into living beings, such as animals or fish includes an injector, such as a syringe, and a transponder support slidably disposed within a barrel of the injector and a transponder slidably disposed within the transponder support. A plunger is provided for causing sliding movement of the transponder within the transponder support. The injector with the associated transponder and transponder support are contained in a sterile package. In use, the injector is removed from the sterile package and a cannula is associated with the transponder.

## Description

### CROSS REFERENCE TO RELATED APPLICATION

This application claims the benefit and priority of U.S. Provisional Application No. 60/361,900 filed February 28, 2002, and entitled COMBINED TRANSPONDER AND INJECTOR, the disclosure of which is incorporated herein by reference.

### BACKGROUND OF THE INVENTION

### 1. Field of the Invention

This invention relates to a method and apparatus for implantation of transponders and, more particularly, to a method and apparatus for implantation of radio frequency transponders into living human beings, such as animals and fish.

### 2. Description of the Related Art

Radio frequency transponders are used to identify animals for a number of purposes, including live stock management and recovery of lost or stolen animals. Prior to use, the transponder is inserted into a cannula and the cannula inserted into an injector. The resultant assembly is then put into a plastic bag and sterilized. In use the assembly is removed from the sterile bag and the transponder implanted either subcutaneously or intramuscularly in the animal.

While such an assembly has generally proven to be satisfactory, it does have a number of disadvantages. First, it requires that the cannula be affixed to the injector. This results in added steps in the manufacture of the assembly, as well as complicates automatic manufacture thereof. Also, in order to accommodate different size animals, for example a kitten and a horse, assemblies with different size cannulas have to be stocked. This results in an increase in inventory with a concomitant increase in cost.

### SUMMARY OF THE INVENTION

The present invention solves the foregoing problems by a system of implantation which separates the cannula from the injector and transponder.

More specifically, in accordance with certain features of the invention, an apparatus for implanting the transponder into living beings, such as animals or fish, includes an injector, such as a syringe, having a barrel, a transponder support in the barrel, a transponder movably disposed with the transponder support and a plunger for moving the transponder within the transponder support.

Preferably the assembly is contained in a sterile plastic bag. In use, the user, for example a veterinarian, will open the sterile package and fix a standard commercially available cannula to the injector.

This system of packaging has a number of advantages over the prior system. Most importantly, it reduces the cost of implantation. Costs are reduced because the cannula does not have to be affixed to the injector and the process of inserting the transponder into the injector can be more readily automated than the prior system. Additionally, the assembly ofthe injector and transponder of the present system is smaller than the prior system and therefore easier to handle, package and ship than the prior system.

Additionally, the injector may have a variable length plunger. This enables the injector to accommodate different size cannulas. As a result, only one size of injector needs to be stocked.

Other features and advantages of the present invention will become apparent from the following description of the invention which refers to the accompanying drawings.

### BRIEF DESCRIPTION OF THE DRAWING(S)

Fig. 1 shows in schematic form a partly cross-sectional view of an assembly of a syringe and transponder in a sterile bag.

Fig. 2 shows the assembly of the injector and transponder of Fig. 1 after being removed from the sterile bag with a cannula affixed thereto.

### DETAILED DESCRIPTION OF PREFERRED EMBODIMENT(S)

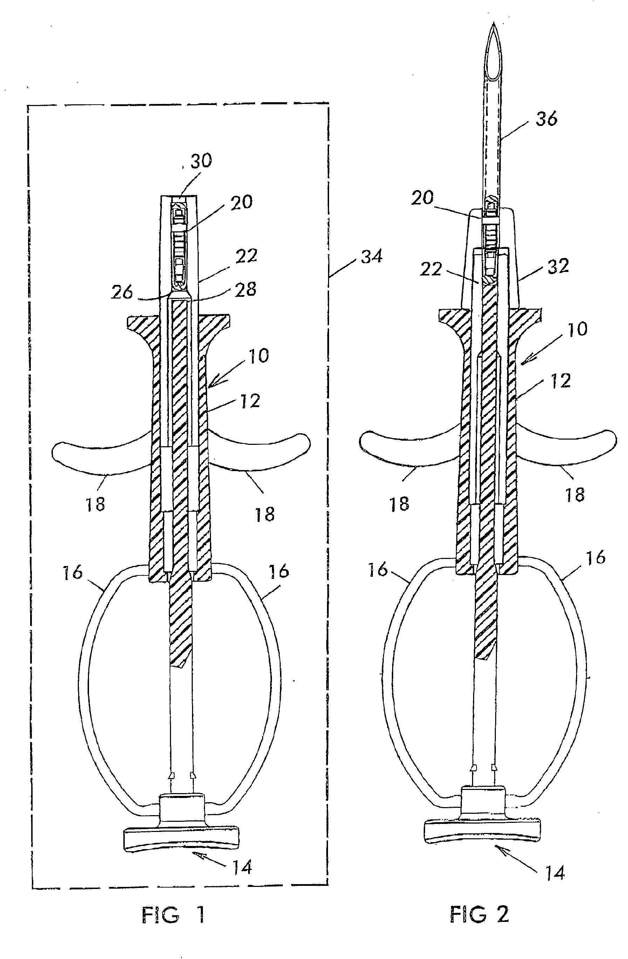

Referring now to Fig. 1, there is shown an injector 10, which includes a barrel 12 and a plunger 14 which is slidable within the barrel 12, the plunger 14 being connected to the barrel 1 by a pair of flexible support elements 16, 16. A pair of finger grips 18, 18 are attached to the outer surface of the barrel to facilitate use of the syringe.

In accordance with the present invention, a radio frequency transponder 20 is slidably disposed within a transponder support 22 which is slidably affixed to the inside of the barrel. 12. The transponder support 22 has a longitudinal bore 24 in which the transponder 20 is located. The bore 24 has a lower opening 26 to allow the leading end 28 of the plunger 14 to contact the transponder 20 and an upper open end 30 to allow the transponder 20 to be ejected from the transponder support 22. The transponder support 22 is externally threaded to receive an internally threaded luer lock 30 (Fig. 2). The external threading of the transponder support 22 and the internal threading of the luer lock 30 are not shown for the purpose of clarity.

The assembly of the injector 10 and transponder 20 are contained in a sterile plastic bag 34. This is done by placing the assembly of the injector 10 and transponder 22 in the plastic bag 34, sealing the bag and then sterilizing the bag 34.

The transponder 20 may be any conventional radio frequency transponder. Generally the transpouder 20 includes a glass or plastic capsule within which is an antenna coil and a microchip which contains a unique identification code. Advantageously, the transponder may be a Datamars T-IS-8010 transponder.

Referring now to Fig. 2, in use, the injector 10 and the transponder 20 are removed from the sterile bag and a standard cannula (needle) 36 affixed to the transponder 20 and to the transponder support 20 with such lock, such as the luer lock 32 such that the leading edge of the transponder 20 is partially located within the bore of the hollow cannula. 36 In order to facilitate this attachment, the transponder support 22 is slid downwardly within the bore of the barrel 12 (from the position shown in Fig. 1 to the position shown in Fig. 2) thereby exposing the leading portion of the transponder 20. This then enables the cannula 3 6 to be placed over the extended portion of the transponder 20.

After the cannula 34 is locked in place by the luer lock 32, the transponder 20 may be implanted into a living being by insertion of the cannula 34 either subcutaneously or intramuscularly into the being by operation of the plunger.

The invention provides a number of advantages over the prior system of implantation including reducing costs. More specifically, eliminating the step of affixing the cannula to the injector. Elimination of this step has the added benefit of facilitating automatic assembly. Finally, because the entire assembly is smaller. handling, packaging and shipping costs are reduced.

An additional advantage results if the plunger is arranged such that its length may be varied. At the present time, the length of the plunger is fixed and this fixed length must be compatible with the length of the cannula. Accordingly, a fixed length plunger is limited to cannulas of the same size. As a result, in order to accommodate different size animals, for example a kitten and a horse, assemblies of different size plungers and corresponding cannulas need to be stocked, thereby resulting in an increase in inventory with a concomitant increase in cost. However, if the injector has a variable length plunger, only a single type injector needs to be stocked. As a result, the number of injectors required to accommodate different size cannulas can be minimized, leading to a substantial savings in inventory cost.

Although the present invention has been described in relation to particular embodiments thereof, many other variations and modifications and other uses will become apparent to those skilled in the art. It is preferred, therefore, that the present invention be limited not by the specific disclosure herein, but only by the appended claims.

## Claims

1. Apparatus for implanting a transponder into a living being which comprises an injector having a barrel, a transponder support within the barrel, a transponder movable within the transponder support and a plunger for causing sliding movement of the transponder within the transponder support.

2. Apparatus in accordance with claim 1, wherein the transponder support is slidable within the barrel and the transponder is slidable within the transponder support.

3. Apparatus in accordance with claim 2, wherein the transponder support has an internal passageway, the transponder is disposed within the internal passageway and the plunger is moveable within internal passageway to cause sliding movement of the transponder within the internal passageway,

4. Apparatus in accordance with claim 3, and further including a lock for locking a caunula to the transponder.

5. Apparatus in accordance with claim 4, wherein the lock comprising a luer lock.

6. Apparatus for implanting a transponder into a living being which comprises a sterile package containing an injector having a barrel, a transponder support movable within the barrel, a transponder movable within the transponder support and a plunger for causing movement of the transponder within the transponder support.

7. Apparatus in accordance with claim 6, wherein the transponder support is slidable within the barrel and the transponder is slidable within the transponder support.

8. Apparatus in accordance with claim 7, wherein the transponder support has an internal passageway, the transponder is disposed within the internal passageway and the plunger is moveable into the internal passageway to cause sliding movement of the transponder within the internal passageway.

9. Apparatus in accordance with claim 8, wherein the injector is a syringe.

10. Apparatus in accordance with any of claim 8, wherein the transponder is a radio frequency transponder.

11. Apparatus for implanting a transponder into a living being, which comprises:
a) a sterile package containing an injector having a barrel, a transponder support disposed within the barrel, a transponder movably disposed within the transponder support and a plunger for causing sliding movement of the transponder within the transponder support;
b) a cannula; and
c) a lock for locking the cannula to the transponder support.

12. Apparatus in accordance with claim 11, wherein the transponder support is slidable within the barrel and the transponder is slidable within the transponder support.

13. Apparatus in accordance with claim 10, wherein the transponder support has an internal passageway, the transponder is disposed within the internal passageway and the plunger is moveable into the internal passageway to cause sliding movement of the transponder within the internal passageway.

14. In a method of implanting a transponder into a living being,
a) providing a sterile package containing an injector and a transponder arranged to be moved by the injector;
b) providing a cannula;
c) removing the injector and transponder from the sterile package; and
d) placing the transponder within the cannula.

15. In a method of implanting a transponder into a living being,
a) providing a sterile package containing an injector having a barrel, a transponder support slidably disposed within the barrel, a transponder slidably disposed within the transponder support and a plunger for causing sliding movement of the transponder within the barrel;
b) providing a cannula;
c) removing the injector and transponder from the sterile package; and
d) attaching the cannula to the transponder support such that the transponder is disposed within the cannula.

16. A method according to claim 13, wherein the injector is a syringe and the transponder is a radio frequency transponder.
